# EUROPÄISCHE PATENTANMELDUNG

(11) **EP 2 058 012 A1**
(43) Veröffentlichungstag der Anmeldung: **13.05.2009**
(21) Anmeldenummer: 07120350.9
(22) Anmeldetag: 09.11.2007
(51) Int. Cl.: A61L 2/00, A61L 2/10

(54) **Vorrichtung zur Virenaktivierung in absorbierenden Flüssigkeiten**

(71) Anmelder: Octapharma AG, 8853 Lachen (CH)
(72) Erfinder: Buchacher, Andrea Dr.,, 1180 Wien (AT); Iberer, Günter Dr.,, 2331 Vösendorf (AT); Karlovits, Manfred, 1110 Wien (AT); Römisch, Jürgen Roland Dr.,, 2440 Gramatneusiedl (AT); Schütz, Raimund, 1220 Wien (AT)
(74) Vertreter: von Kreisler Selting Werner

(57) **Zusammenfassung**

Eine Vorrichtung zur Vireninaktivierung in absorbierenden Flüssigkeiten weist einen Rotor (10) und ein den Rotor (10) umgebendes Gehäuse (12) auf. Hierdurch ist zwischen dem Rotor (10) und dem Gehäuse (12) ein Spalt ausgebildet, durch den die zu behandelnde Flüssigkeit geleitet wird. Innerhalb des Rotors (10) ist eine Strahlungserzeugungseinrichtung (50) zur Erzeugung von vireninaktivierender Strahlung angeordnet, Zumindest ein Teil der Mantelfläche (14) des Rotors (10) ist aus strahlungsdurchlässigem Material wie Quarzglas hergestellt.

## Beschreibung

Die Erfindung betrifft eine Vorrichtung zur Vireninaktivierung in absorbierenden Flussigkeiten, wie insbesondere Blutplasma und daraus gewonnenen Produkten/Konzentraten.

Bei aus Blutplasma hergestellten Medikamenten besteht das Risiko, dass in dem Blutplasma vorhandene Viren auch in den Medikamenten enthalten sind. Durch Spender- und Plasma-Mustertestungen kann das Risiko verringert werden. Ferner sind unterschiedliche Abreicherungsmethoden bekannt, um eine möglichst hohe Virussicherheit zu erzielen. Bisher ist jedoch noch keine Methode bekannt, mit der es insbesondere im industriellen Maßstab möglich ist, alle unterschiedlichen Arten von Viren effektiv zu inaktivieren. Insbesondere kleine und nicht lipidumhüllte Viren können nur schwer inaktiviert werden.

Zur Vireninaktivierung ist unter anderem der Einsatz von UVC-Strahlung bekannt. Die UVC-Strahlung einer Wellenlänge von ca. 254 nm wirkt inaktivierend auf die RNA und/oder DNA der Viren und ist insbesondere bei Viren mit kleiner RNA und/oder DNA besonders wirkungsvoll. UVC-Strahlung kann sowohl auf nicht umhüllte als auch auf umhüllte Viren angewandt werden. Sie ist insbesondere zur Vireninaktivierung von kleinen nicht lipidumhullten Viren gut geeignet.

Bei Produkten wie Medikamenten, die aus Blutplasma gewonnen werden, handelt es sich meist um Proteinlösungen. Diese haben beim Einsatz von UVC-Strahlung den Nachteil, dass ein Teil der eingestrahlten Strahlung absorbiert wird. Dies fuhrt zur Abschwächung der Intensität der Strahlung. Je konzentrierter die Proteinlösung ist, desto geringer wird die Transmission der Lösung. Die Strahlung dringt somit nur geringfügig tief in die Proteinlösung ein und ist somit bereits stark abgeschwächt, bevor sie auf Viren trifft. Dies führt dazu, dass nicht sämtliche Viren zuverlässig inaktiviert werden können.

Bei den meisten bekannten Methoden zur Inaktivierung von Viren mittels UVC-Strahlung ist es somit erforderlich, dass die Vorrichtungen mit niedrigen Flussraten betrieben werden und die optische Trübung der zu behandelnden Lösung niedrig ist. Dies fuhrt bei der Virusinaktivierung durch UVC-Strahlung von Blutprodukten dazu, dass das Ausgangsmaterial sehr stark verdünnt werden muss. Vier- bis zehnfache Verdünnungen sind erforderlich. Bei industrieller Herstellung eines Blutprodukts von beispielsweise 1000 Litern wurde aufgrund der Verdünnung und der geringen Flussrate die Virusinaktivierung mit UVC-Strahlung mehrere Tage in Anspruch nehmen.

Aus US 6,576,201 ist es bekannt, zwei konzentrische Zylinder vorzusehen, so dass zwischen den beiden Zylindern ein Spalt ausgebildet ist. Die zu bestrahlende Flussigkeit wird durch diesen Spalt geleitet. Zur Bestrahlung der Flussigkeit ist der äußere Zylinder mit Strahlungsquellen umgeben und aus Quarzglas hergestellt. Die UVC-Strahlung kann durch das für diese Strahlung transparente Quarzglas in dem Bereich zwischen den beiden Zylindern eindringen und auf die Flussigkeit einwirken. Zusätzlich wird der innere Zylinder in eine Rotationsbewegung versetzt. Dies führt dazu, dass die Strömung in dem Spalt zusätzlich durchmischt wird. Es tritt innerhalb des Spaltes die sogenannte Taylor-Couette-Strömung auf. Durch die Drehung des inneren Zylinders wird somit eine Sekundärströmung (Taylor-Couette-Strömung) in dem Spalt erzeugt. Dies führt dazu, dass die Flüssigkeit mit großer Wahrscheinlichkeit über die Länge der beiden Zylinder einen Bereich durchströmt, in den noch entsprechend starke UVC-Strahlung eindringt und eine Inaktivierung der Viren hervorgerufen werden kann. Der Bereich, in den die UVC-Strahlung eindringt und noch eine hohe Intensität aufweist, ist die unmittelbar an die Innenseite des äußeren Zylinders angrenzende Schicht.

Zur industriellen Herstellung von Produkten, wie Medikamenten aus Blutplasma, ist es erforderlich, eine Vorrichtung, in der eine sekundäre Strömung auftritt, beispielsweise eine Taylor-Courette Strömung, mit höheren Durchflussraten zu konstruieren. Um die Exposition der Flüssigkeit mit der UVC-Strahlung gleich lang zu halten und damit eine gute Vireninaktivierung zu ermöglichen, besteht entweder die Möglichkeit, den Zylinderdurchmesser zu vergrößern oder die Zylinder zu verlängern. Unabhängig davon, dass entsprechend lange Zylinder aus Quarzglas schwierig herzustellen und teuer sind, wurde eine derartige Vorrichtung einen großen Bauraum einnehmen. Insbesondere musste eine Vorrichtung mit einem entsprechend langem Zylinder horizontal angeordnet werden, da ansonsten die üblichen Raumhöhen etc. zu gering wären. Eine weitere Möglichkeit besteht darin, mehrere Zylinder hintereinander anzuordnen, wobei es sich auch hierbei um eine technisch aufwendige und einen großen Bauraum beanspruchende Lösung handelt. Eine Vergrößerung des Durchmessers des äußeren aus Quarzglas hergestellten Zylinders ist technisch nur begrenzt möglich, da Quarzglasrohre mit der gewunschten Genauigkeit bei der Materialqualität sowie in den Abmessungen nur mit limitiertem Durchmesser hergestellt werden können.

Ferner muss bei der Konstruktion einer Vorrichtung zur Vireninaktivierung mit UVC-Strahlung berucksichtigt werden, dass um die Nativität der Proteine sicherzustellen, eine gleichmäßige und schonende Bestrahlung gewährleistet sein muss.

Aufgabe der Erfindung ist es, eine Vorrichtung zur effektiven Virenabreicherung in absorbierenden Flüssigkeiten zu schaffen, mit der hohe Flussraten erzielt werden können, so dass insbesondere eine Virenabreicherung im industriellen Maßstab möglich ist.

Die Lösung der Aufgabe erfolgt erfindungsgemäß durch die Merkmale des Anspruchs 1.

In besonders bevorzugter Ausführungsform erfolgt die Lösung der Aufgabe durch
1. eine effektivere Nutzung der Strahlungsenergie, durch Anordnung der Strahlungsquelle innerhalb des Rotors;
2. einen höheren Durchmesser des Gerätes und damit verbunden ein höheres Bestrahlungsvolumen, um die erforderliche mittlere Verweilzeit der Flüssigkeit im strahlungsdurchlässigen Teil des Reaktors zu erreichen;
3. Verdopplung der Bestrahlungsfläche sofern beide, insbesondere zylindrische Gehäuseteile strahlungsdurchlässig sind.

Die erfindungsgemäße Vorrichtung zur Vireninaktivierung bzw. Virenabreicherung in absorbierenden Flüssigkeiten weist einen Rotor auf, der von einem Gehäuse umgeben ist. Hierdurch ist zwischen dem insbesondere zylindrisch ausgebildeten Rotor und dem ebenfalls vorzugsweise zylindrisch ausgebildeten, insbesondere konzentrisch zum Rotor angeordneten Gehäuse ein Spalt ausgebildet. Der Spalt weist vorzugsweise eine Breite von 0,1 bis 5,0 cm, bevorzugt von 0,5 bis 2,0 cm auf. Die zu behandelnde Flussigkeit wird durch den Spalt zwischen Rotor und Gehäuse hindurch geleitet. Dies kann durch Pumpen oder durch eine Druckdifferenz erfolgen. Zur Vireninaktivierung ist ferner eine Strahlungserzeugungseinrichtung vorgesehen, die entsprechend der zur Vireninaktivierung geeignete Strahlung erzeugt. Bei der Strahlung handelt es sich insbesondere um UV-Strahlung im Bereich von 200 bis 300 nm. Besonders bevorzugt ist Strahlung im Bereich von 230 bis 270 nm und insbesondere UVC-Strahlung mit einer bevorzugten Wellenlänge von ca. 254 nm. Erfindungsgemäß ist die Strahlungserzeugungseinrichtung innerhalb des Rotors angeordnet. Die erzeugte Strahlung tritt somit durch einen strahlungsdurchlässigen Teil des Rotors hindurch und dringt von innen in die zu behandelnde Flüssigkeit ein. Das Gehäuse kann somit aus strahlungsundurchlässigem Material hergestellt sein. Es ist erfindungsgemäß möglich einen Rotor, bei dem es sich in bevorzugter Ausführungsform um einen Zylinder aus Quarzglas handelt, mit einem maximalem Durchmesser vorzusehen. Derzeit sind Zylinder aus Quarzglas mit ausreichender Qualität und Genauigkeit nur mit einem limitierten Durchmesser herstellbar. Da im Unterschied zum Stand der Technik der äußere Zylinder bzw. das Gehäuse nicht aus Quarzglas hergestellt sein muss, kann der Durchmesser der Vorrichtung vergrößert werden. Dies fuhrt bei gleicher Spaltbreite zur Vergrößerung des Bestrahlungsvolumens, das heißt bei gleichbleibender mittlerer Verweilzeit der Flussigkeit im strahlungsdurchlässigen Teil des Reaktors erhöht sich die Flussrate.

Da die Strahlungserzeugungseinrichtung erfindungsgemäß innerhalb des Rotors angeordnet ist, besteht ferner der Vorteil, dass im Wesentlichen die gesamte von der Strahlungserzeugungseinrichtung erzeugte Strahlung nach außen durch die Rotorwand in die zu behandelnde Flüssigkeit eindringt. Das Vorsehen von Reflektoren, wie es bei außerhalb des Gehäuses bzw. außerhalb des äußeren Zylinders angeordneten Strahlungserzeugungseinrichtungen erforderlich ist, ist erfindungsgemäß nicht notwendig. Die durch die Strahlungserzeugungseinrichtung erzeugte Energie kann somit effektiver genutzt werden, wodurch die Flussraten entsprechend erhöht werden können. Erfolgt die Bestrahlung der Flussigkeit durch den äußeren Zylinder, sind für eine gleichmäßige Bestrahlung rund um den Reaktor vorzugsweise mehrere Strahlungsquellen mit gleichmäßigem Abstand zueinander vorgesehen.

Bei gleicher Lampenanzahl steigt mit zunehmendem Durchmesser auch der Abstand zwischen den Lampen. Werden die Lampen hingegen innerhalb des Rotors angeordnet, kann der Abstand zwischen den Lampen beliebig verringert werden. Hierdurch ist eine gleichmäßige Behandlung der Flüssigkeit mit Strahlung möglich. Bei kleineren Geräten kann die Strahlungsquelle auch aus nur einer Lampe bestehen.

Durch das erfindungsgemäße Vorsehen der Strahlungserzeugungseinrichtung innerhalb des Rotors kann insbesondere aufgrund der möglichen Vergrößerung des Durchmessers des inneren Rotors und der besseren Strahlungsausbeute eine höhere Durchflussrate realisiert werden. Die erfindungsgemäße Vorrichtung ist insbesondere fur Flussraten von mehr als 150 Liter pro Stunde, insbesondere mehr als 300 Liter pro Stunde geeignet. In Abhängigkeit der zu behandelnden Flussigkeit können sogar Durchflussraten von 500 bis 1000 oder sogar mehr als 1000 Liter pro Stunde realisiert werden.

Die Spaltbreite, das heißt der Abstand zwischen der Außenseite des Rotors und der Innenseite des Gehäuses ist vorzugsweise radial konstant. Dies kann dadurch erzielt werden, dass sowohl der Rotor als auch das Gehäuse als Zylinder ausgebildet sind, die konzentrisch angeordnet sind. Zumindest sollte das Gehäuse eine zylindrische Bohrung aufweisen in der der Rotor angeordnet ist. Vorzugsweise ist die Spaltbreite auch über die Länge des Zylinders konstant. In Abhängigkeit der zu bestrahlenden Flussigkeit beträgt die Spaltbreite 0,1 bis 5,0 cm, bevorzugt 0,5 bis 2,0 cm. Die Spaltbreite ist hierbei insbesondere derart gewählt, dass aufgrund der Rotation des Rotors innerhalb des Spalts eine Sekundärströmung, insbesondere eine Verwirbelung der Flussigkeit erfolgt. Die Sekundärströmung kann eine Taylor-Couette-Strömung sein.

Vorzugsweise ist die gesamte Mantelfläche des Rotors im Bereich des Spalts, in dem die Sekundärströmung auftritt, strahlungsdurchlässig. Bei einer besonders bevorzugten Ausführungsform handelt es sich somit um einen kreisringförmigen Zylinder aus Quarzglas. Wenngleich Quarzglas besonders geeignet ist, ist es für die Erfindung wesentlich, dass die von der Strahlungserzeugungseinrichtung ausgesendete Strahlung, bei der es sich insbesondere um ultraviolettes Licht, besonders bevorzugt um UVC-Licht handelt, möglichst ungeschwächt durch den entsprechenden Bereich, das heißt insbesondere durch die Mantelfläche des Rotors hindurchdringen kann.

Die in bevorzugter Ausführungsform stationär innerhalb des Rotors angeordnete Strahlungserzeugungseinrichtung kann eine oder mehrer Strahlungsquellen aufweisen. Hierbei kann es sich um eine zylindrische Strahlungsquelle oder mehrere insbesondere auf einer Kreislinie angeordnete Strahlungsquellen wie UVC-Strahler handeln.

In Abhängigkeit der zu behandelnden Flüssigkeit besteht bei der erfindungsgemäßen Vorrichtung ferner die Möglichkeit, die Strahlungsintensität, beispielsweise aufgrund der Anzahl und/oder der Eigenschaften der Strahlungsquellen zu variieren. Ferner ist es möglich, beim Vorsehen mehrerer Strahlungsquellen diese unterschiedlich anzusteuern.

In besonders bevorzugter Ausführungsform ist der Rotor über eine Hohlwelle gelagert, Hierbei kann die Lagerung einseitig oder auf beiden Seiten erfolgen, wobei der insbesondere zylindrisch ausgebildete aus Quarzglas hergestellte Rotor mit zwei Deckeln verschlossen ist. Vorzugsweise sind die eine oder beide Hohlwellen jeweils mit einem Deckel verbunden. Da die zumindest eine Rotorwelle als Hohlwelle ausgebildet ist, ist es möglich, die Energieleitungen, das heißt die Stromleitung zu der Strahlungserzeugungsquelle durch die Hohlwelle zu fuhren. Ferner kann durch die Hohlwelle eine Halterung geführt werden, die ein stationäres Halten der Strahlungseinrichtung gewährleistet. Um ein Überhitzen der Lampe zu vermeiden, kann der Innenraum des Rotors gekuhlt werden. Das Vorsehen zweier Wellen zur Lagerung des Rotors hat ferner den Vorteil, dass eine sehr exakte Lagerung des Rotors möglich ist, so dass der Wartungsaufwand reduziert werden kann. Ferner ist auch über die Länge des Zylinders eine sehr exakte konstante Spaltbreite einfacher realisierbar.

Das Gehäuse weist in bevorzugter Ausführungsform zwei Deckel auf, wobei die mindestens eine Hohlwelle durch den oder die Deckel geführt ist. Die Lagerung des Rotors erfolgt hierbei vorzugsweise in den Gehäusedeckeln. Die Flüssigkeit kann über eine oder mehrere Zuführöffnungen in den Reaktor gelangen. Die Zufuhröffnungen können sich am Gehäusedeckel oder an der Gehäusewand befinden. Auch kann die Flüssigkeit bereits eine Strömung in Drehrichtung des Rotors besitzen, bevor sie den Spalt erreicht. Diese Strömung kann durch entsprechende Anordnung der Zufuhröffnungen bzw. Einbauten im Behälterboden hervorgerufen werden. Das Fluid verlässt den Reaktor über eine oder mehrere Abfuhröffnungen am anderen Ende des Reaktors. So wie die Zufuhröffnungen können sich die Abführöffnungen am Gehäusedeckel oder am Gehäusemantel befinden. Zur Verstärkung der Sekundärströmung im Spalt ist es ferner möglich, das Gehäuse bzw. den äußeren Zylinder rotierend auszugestalten, wobei die Drehrichtung des äußeren Zylinders vorzugsweise entgegen der Drehrichtung des Rotors wäre.

In bevorzugter Ausführungsform ist die als Hohlwelle ausgebildete Lagerwelle des Rotors gleichzeitig als Antriebswelle ausgebildet. Der Antrieb erfolgt beispielsweise über einen Elektromotor unter Zwischenschaltung eines Getriebes und/oder durch Vorsehen eines Zahnriemens oder dergleichen. Über eine Steuereinrichtung, die in den Rotor integriert sein kann, wird eine gleichmäßige, konstante Umdrehungszahl des Rotors gewährleistet. Vorzugsweise ist die Drehzahl des Motors regelbar, um die Rotorgeschwindigkeit an die Flussigkeitseigenschaften anpassen zu können. Beispielsweise ist eine Anpassung an die Flussigkeitseigenschaften, wie die Viskosität, die optische Trübe etc. vorteilhaft. Hierzu ist in einer bevorzugten Weiterbildung der Erfindung eine Messeinrichtung zur Messung zusätzlicher Parameter vorgesehen. Die von der Messeinrichtung gemessenen Flussigkeitseigenschaften können an eine Steuereinrichtung übermittelt werden. Durch die Steuereinrichtung kann die Flussrate in Abhängigkeit der gemessenen Flussigkeitseigenschaften angepasst werden. Hierzu ist die Steuereinrichtung beispielsweise auch mit der Pumpe, durch die die Flüssigkeit durch den Spalt der Vorrichtung gepumpt wird, verbunden. Anstatt des Vorsehen einer Pumpe kann auch eine Druckdifferenz zwischen Zuführöffnung und Abführöffnung erzeugt werden, um ein Fliessen der Flüssigkeit hervorzurufen.

Bei einer weiteren Ausführungsform der Erfindung ist eine zweite Strahlungserzeugungseinrichtung zusätzlich zu der innerhalb des Rotors vorgesehenen Strahlungserzeugungseinrichtung vorgesehen. Die zweite Strahlungserzeugungseinrichtung ist außerhalb des Gehäuses bzw. des äußeren Zylinders angeordnet. Hierzu ist zumindest ein Teil des Gehäusemantels strahlungsdurchlässig, insbesondere aus Quarzglas hergestellt, Aufgrund des Vorsehens einer zweiten Strahlungserzeugungseinrichtung ist es möglich, die Bestrahlungsoberfläche zu vergrößern. Hierdurch kann die Durchflussrate weiter erhöht werden. Es ist möglich, einen Teil des Gehäuses als Zylinder aus strahlungsdurchlässigem Material auszubilden. Ebenso ist es möglich, nur einzelne Bereiche entsprechend Fenstern aus strahlungsdurchlässigem Material, insbesondere Quarzglas herzustellen. Das Vorsehen nur einzelner Bereiche bzw. Fenster aus entsprechendem Material insbesondere Quarzglas hat den Vorteil, dass die technische Beschränkung des maximalen Durchmessers eines Quarzglaszylinders nicht gegeben ist.

Die zweite Strahlungserzeugungseinrichtung weist vorzugsweise mehrere Strahlungsquellen auf, die das Gehäuse vorzugsweise vollständig umgeben. Die Strahlungsquellen können mit Reflektoren versehen sein.

Besonders bevorzugt ist die Verwendung der erfindungsgemäßen Vorrichtung zur Bestrahlung von Flüssigkeiten wie biologischen Flüssigkeiten, die auch rekombinante/transgene Proteine enthalten können, therapeutischen Flüssigkeiten, Humanplasma, proteinhaltige Flüssigkeiten und Flüssigkeiten mit anderen Biopolymeren. Die Vorrichtung ist auch zur Sterilisierung von Flüssigkeiten geeignet. Insbesondere ist die Vorrichtung zur Bestrahlung von aus Plasma hergestellten oder herzustellende Flüssigkeiten geeignet, wie zum Beispiel Plasma und/oder Fraktionen enthaltend Immunglobuline, Fibrinogen, F VIII/vWP, F IX, Prothrombinkomplex, Albumin, A1AT, AT III, Plasminogen, Transferrin, Fibronektin, Growth Factors.

Wenngleich die Vorrichtung unter Verwendung einer oder mehrerer Strahlungserzeugungseinrichtungen, die UV- insbesondere UVC-Strahlungen erzeugen, zur Vireninaktivierung bevorzugt ist, kann insbesondere bei Verwendung anderer Strahlung mit Hilfe der Vorrichtung auch eine Inaktivierung von Bakterien, Mirkoorganismen und dergleichen erfolgen.

Nachfolgend wird die Erfindung anhand bevorzugter Ausführungsformen näher erläutert.

### Es zeigen:

- Fig. 1: eine schematische Schnittansicht einer ersten bevorzugten Ausführungsform der Vorrichtung, und
- Fig. 2: eine schematische Schnittansicht einer zweiten bevorzugten Ausführungsform der Vorrichtung.

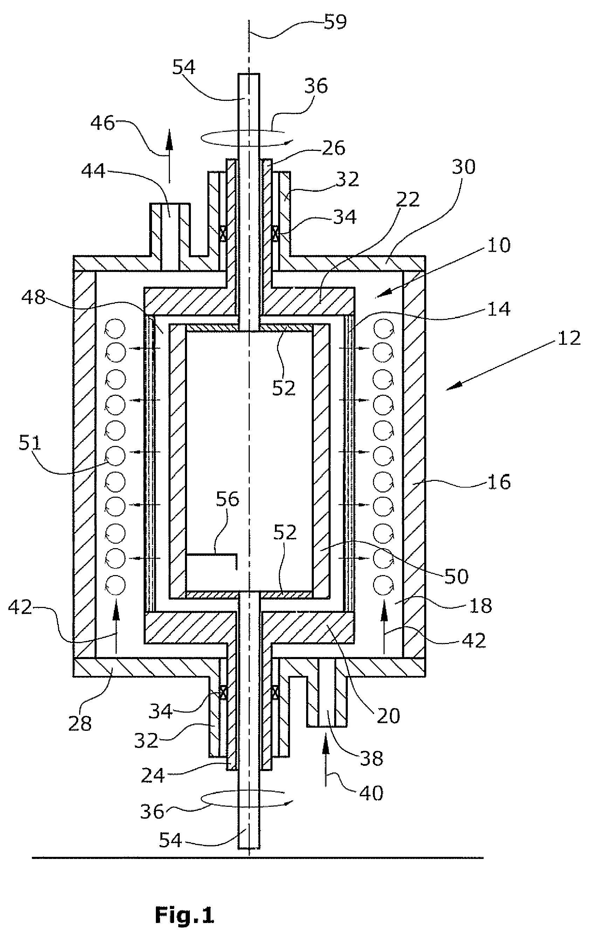

Die in Fig. 1 stark vereinfacht schematisch dargestellte erste Ausführungsform der erfindungsgemäßen Vorrichtung zur Vireninaktivierung in absorbierenden Flüssigkeiten weist einen zylindrischen Rotor 10 auf, der von einem ebenfalls zylindrischen Gehäuse 12 umgeben ist. Zwischen dem kreiszylindrischen rohrförmigen Teil 14 des Rotors 10 und dem vorzugsweise ebenfalls kreisringförmigen zylindrischen Gehäuseteil 16 des Gehäuses 12 ist ein Spalt 18 ausgebildet.

Der kreisringzylindrische Teil 14 des Rotors 10 ist mit einem ersten Deckel 20 sowie einem zweiten Deckel 22 verschlossen. Die beiden Deckel 20, 22 sind jeweils fest mit einer Hohlwelle 24, 26 verbunden.

Das kreisringzylindrische Teil 16 des Gehäuses 12 ist mit einem ersten Deckel 28 und einem zweiten Deckel 30 verschlossen. Die beiden Deckel 28, 30 weisen jeweils einen zylindrischen Ansatz 32 auf. Die zylindrischen Ansätze 32 umgeben im dargestellten Ausführungsbeispiel die Hohlwellen 24, 26. Zwischen den Hohlwellen 24, 26 und den zylindrischen Ansätzen 32 sind Lager und Dichtungen 34 vorgesehen.

Eine der beiden Hohlwellen 24, 26 ist ggf, über ein zwischengeschaltetes Getriebe, einen Zahnriemen oder dergleichen mit einer Antriebseinrichtung wie einem Elektromotor verbunden. Hierdurch kann der Rotor 10 entsprechend eines Pfeils 36 gedreht werden. Durch Rotation des Rotors 10 in Richtung des Pfeile 36 werden in dem Spalt durch Pfeile 51 schematisch dargestellte Sekundärströmungen erzeugt. Es handelt sich hierbei um sogenannte Taylor-Couette-Strömungen.

Der erste Deckel 28 des Gehäuses 12 weist eine Zuführöffnung 38 zum Zufuhren der zu behandelnden Flüssigkeit in Richtung eines Pfeils 40 auf. Die Flüssigkeit strömt sodann in Richtung der Pfeile 42 durch den zwischen dem Rotor 10 und dem Gehäuse 12 ausgebildeten kreisringförmigen Spalt und tritt sodann durch eine im zweiten Deckel 30 vorgesehene Auslassöffnung 44 in Richtung eines Pfeils 46 aus der Vorrichtung aus.

Erfindungsgemäß ist der Rotor 10 hohl ausgebildet, so dass in einem Innenbereich 48 eine Strahlungserzeugungseinrichtung 50 angeordnet werden kann. Die Strahlungserzeugungseinrichtung 50, die ggf. mehrere Strahlungsquellen aufweist, wird von Halterungen 52 getragen. Zur Energieversorgung der Strahlungserzeugungseinrichtung 50 können Leitungen 56 durch die hohlen Ansätze 54 nach außen geführt werden.

Die Mantelfläche 14 bzw. der kreisringzylindrische Teil 14 des Rotors 10 ist vorzugsweise aus Quarzglas hergestellt. Hierdurch ist es möglich, wie durch die Pfeile angedeutet, dass die von der Strahlungserzeugungseinrichtung 50 abgegebene Strahlung, insbesondere UVC-Strahlung in den Spalt 18 eindringt und das in dem Spalt 18 vorhandene Medium zu bestrahlt. Da es sich bei dem Medium insbesondere um eine Strahlung absorbierende Flüssigkeit handelt, die aufgrund der Drehung des Rotors 10 verwirbelt ist, kann eine gute Vireninaktivierung gewährleistet werden.

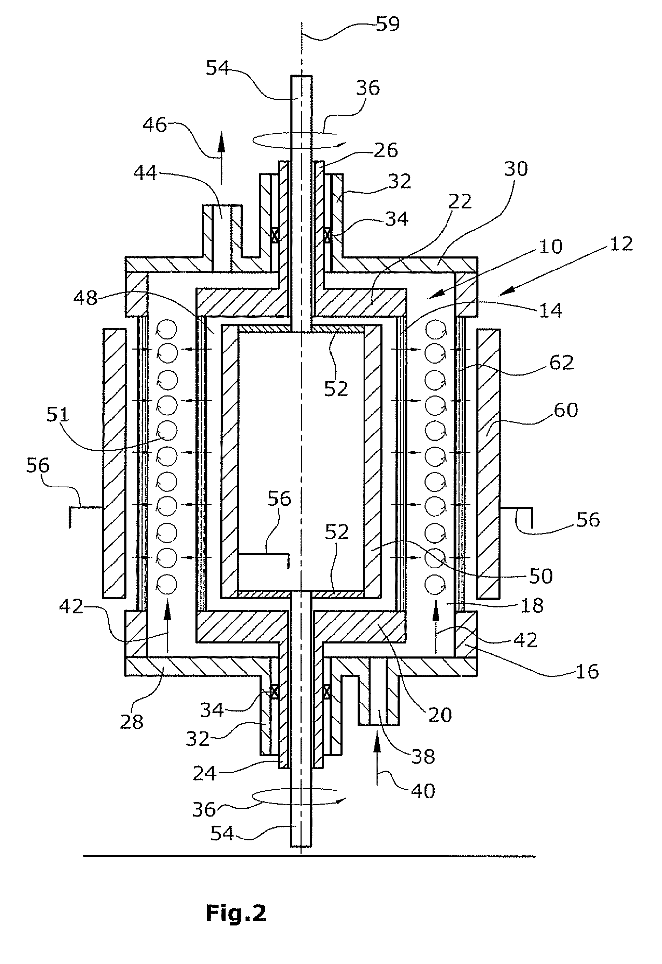

Bei einer zweiten bevorzugten Ausführungsform der Erfindung (Fig. 2) sind ähnliche oder identische Bauteile mit denselben Bezugszeichen gekennzeichnet.

Die zweite Ausführungsform der Erfindung weist zusätzlich eine zweite Strahlungserzeugungseinrichtung 60 auf, die außerhalb des Gehäuses 12 angeordnet ist. Die äußere zylindrische Mantelfläche des Gehäuses 12 ist somit im dargestellten Ausführungsbeispiel als Zylinder 62 aus Quarzglas hergestellt. Der Zylinder 62 erstreckt sich über die selbe Länge wie der innere Zylinder 14 des Rotors 10. Ggf. können außerhalb der zweiten Strahlungserzeugungseinrichtung 60, die auch aus mehreren Strahlungsquellen aufgebaut sein kann, zusätzlich Reflektoren vorgesehen sein. Die in dem Spalt 18 vorgesehene Flüssigkeit wird somit sowohl von innen als auch von außen bestrahlt. Hierdurch kann die Spaltbreite vergrößert und/oder die Durchflussrate erhöht werden.

Der Rotor 10 sowie das Gehäuse 12 sind vorzugsweise in beiden Ausführungsformen konzentrisch zu einer Mittelachse 59 angeordnet. Es handelt sich jeweils um zur Achse 59 rotationssymmetrische Bauteile, die koaxial zueinander angeordnet sind. Lediglich die Zuführöffnung 38 und die Abfuhröffnung 44 bzw. die entsprechenden zylindrischen Stutzen an den Deckeln 28, 30 sind nicht rotationssymmetrisch ausgebildet.

## Patentansprüche

1. Vorrichtung zur Vireninaktivierung in absorbierenden Flüssigkeiten, mit
einem Rotor (10) und
einem den Rotor (10) umgebenden Gehäuse (12) zur Ausbildung eines Spalts (18) zur Hindurchleitung der Flüssigkeit,
**dadurch gekennzeichnet, dass**
innerhalb des Rotors (10) eine Strahlungserzeugungseinrichtung (50) angeordnet ist, wobei zumindest ein Teil einer Mantelfläche (14) des Rotors (10) aus strahlungsdurchlässigem Material besteht.

2. Vorrichtung nach Anspruch 1, **dadurch gekennzeichnet, dass** die
Strahlungserdzeugungseinrichtung (50) vireninaktivierende Strahlung erzeugt.

3. Vorrichtung nach Anspruch 1 oder 2, **dadurch gekennzeichnet, dass** die
Strahlungserzeugungseinrichtung (50) UVC-Strahlung, besonders bevorzugt in einem Wellenlängenbereich von 200 bis 280 nm, insbesondere 250 bis 260 nm, und besonders bevorzugt 254 nm erzeugt.

4. Vorrichtung nach einem der Ansprüche 1 bis 3, **dadurch gekennzeichnet, dass** die Breite des Spalts (18) in Abhängigkeit der zu bestrahlenden Flussigkeit derart gewählt ist, dass innerhalb des Spaltes (18) eine Sekundärströmung (51) entsteht.

5. Vorrichtung nach einem der Ansprüche 1 bis 4, **dadurch gekennzeichnet, dass** die Mantelfläche (14) im Bereich des Spaltes (18), in dem die Sekundärströmung (51) auftritt, strahlungsdurchlässig ist.

6. Vorrichtung nach einem der Ansprüche 1 bis 5, **dadurch gekennzeichnet, dass** die Strahlungserzeugungseinrichtung (50) stationär oder beweglich angeordnet ist.

7. Vorrichtung nach einem der Ansprüche 1 bis 6, **dadurch gekennzeichnet, dass** die Strahlungserzeugungseinrichtung (50) eine oder mehrere, dann insbesondere auf einer Kreislinie angeordnete Strahlungsquellen aufweist.

8. Vorrichtung nach einem der Ansprüche 1 bis 7, **dadurch gekennzeichnet, dass** der Rotor (10) mit einer Antriebseinrichtung verbunden ist, durch die der Rotor (10) insbesondere mit konstanter Drehzahl angetrieben wird.

9. Vorrichtung nach einem der Ansprüche 1 bis 8, **gekennzeichnet durch** eine Messeinrichtung, wobei vorzugsweise über eine Steuereinrichtung die Flussrate ggf. zusätzlich in Abhängigkeit von Flussigkeitseigenschaften und der gemessenen Strahlungsintensität angepasst ist.

10. Vorrichtung nach einem der Ansprüche 1 bis 9, **dadurch gekennzeichnet, dass** die Messeinrichtung zur Bestimmung der Rotordrehzahl und/oder Flussigkeitstemperatur geeignet ist.

11. Vorrichtung nach einem der Ansprüche 1 bis 10, **gekennzeichnet durch** eine zweite außerhalb des Gehäuses (12) und/oder im Gehäusemantel angeordnete zweite Strahlungserzeugungseinrichtung (60), wobei zumindest ein Teil des Gehäuses (12) strahlungsdurchlässig ist.

12. Verwendung der Vorrichtung nach einem der Ansprüche 1 bis 11 zur Bestrahlung von Flüssigkeiten wie biologischen Flüssigkeiten, therapeutischen Flüssigkeiten, Humanplasma, proteinhaltigen Flussigkeiten und/oder Flüssigkeiten mit Biopolymeren.

13. Verwendung nach Anspruch 12, wobei die Flüssigkeiten Plasma und/oder Fraktionen enthaltend Immunglobuline, Fibrinogen, FV
III/vWF, F IX, Prothrombinkomplex, Albumin, A1AT, AT III, Plasminogen, Transferrin, Fibronektin, Growth Factors oder Kombinationen davon sind.

14. Verwendung der Vorrichtung nach Anspruch 12 oder 13 zur
Inaktivierung von Viren, Bakterien und/oder Mikroorganismen in Flussigkeiten, insbesondere in absorbierenden Flüssigkeiten.
